# EUROPEAN PATENT APPLICATION

(11) **EP 1 199 562 A1**
(43) Date of publication of application: **24.04.2002**
(21) Application number: 01917849.0
(22) Date of filing: 06.04.2001
(51) Int. Cl.: G01N 30/88, G01N 30/14

(54) **METHOD FOR MEASURING CONCENTRATIONS AND MOLECULAR WEIGHTS OF GLUE AND GELATIN**

(30) Priority: 29.05.2000 JP 2000158938
(71) Applicant: Mitsui Mining & Smelting Co., Ltd., Tokyo 141-8584 (JP)
(72) Inventor: TAGUCHI, Takeo, Mitsui Mining & Smelting Comp. Ltd, Ageo-shi, Saitama 362-0021 (JP); KOMODA, Yasuo, Mitsui Mining & Smelting Comp., Ltd, Ageo-shi, Saitama 362-0021 (JP); OKADA, Kenzo, Urawa-shi, Saitama 336-0021 (JP); HATA, Hiroshi, Mitsui Mining & Smelting Comp., Ltd, Ageo-shi, Saitama 362-0021 (JP); KURIHARA, Miho, Mitsui Mining & Smelting Comp. Ltd, Ageo-shi, Saitama 362-0021 (JP)
(74) Representative: Forstmeyer, Dietmar, Dr. rer. nat., Dipl.-Chem.
(86) International application number: JP0102995
(87) International publication number: WO0192869

(57) **Abstract**

A method of measuring a concentration and a molecular weight distribution of glue or gelatin contained in an electrolyte, which comprises high performance liquid chromatography combined with column switching.

## Description

### BACKGROUND OF THE INVENTION

### Field of the Invention:

The present invention relates to a method of measuring a concentration and a molecular weight distribution of glue or gelatin present in solution in a small amount, particularly a copper electrolyte. The present invention more particularly relates to a method which makes it possible to measure the concentration and molecular weight distribution of components whose molecular weights are as low as 2500 or less.

### Description of the Related Art:

An electrolyte used in electrolytic refining (electrorefining) of crude copper or copper foil production often contains a small amount of glue or gelatin (hereinafter sometimes inclusively referred to as glue) as an additive. Glue in the electrolyte serves to control the physical properties, such as mechanical strength, surface crystal structure, roughness, and hardness, of electrodeposited copper, e.g., copper foil. It is of great importance to control a glue concentration in the electrolyte in order to manufacture a product of stable quality.

It is said that the molecular size of glue is influential on copper electrodeposition. Further, glue decomposes very rapidly into low molecular components by the action of high-concentration sulfuric acid and the electrolytic action. For these reasons, it is also important to know the molecular weight distribution of glue.

Methods heretofore proposed for measuring the concentration of a small amount of glue in a copper electrolyte include electrochemical techniques, such as a method comprising measuring the polarization (see, for example, Japanese Patent Laid-Open No. 8-304338) and a method comprising precipitating a small amount of copper on a rotating electrode and dissolving the copper, and a dye adsorption method comprising collecting glue on filter paper, staining the glue with a dye, and measuring the absorbance (see, for example, Japanese Patent Laid-Open No. 6-337247).

Electrochemical methods such as the one disclosed in the 8-304338, however, are susceptible to the influences of co-existing substances. The dye adsorption method as taught in the 6-337247 is disadvantageous in that glue having a molecular weight of 20,000 or smaller cannot be collected quantitatively. Further, none of the heretofore proposed methods furnishes information about the molecular weight distribution of glue. Moreover, many of the conventional measuring methods have been carried out in the co-presence of an electrolyte component, or even where a sample is pretreated to remove the electrolyte component before measurement, such a pretreatment is time-consuming, and it is very likely that glue decomposition proceeds in the meantime. Therefore, the methods cannot be seen as successful in accurately evaluating the state of glue under analysis.

It has been considered difficult to measure low molecular weight components of glue under the influences of copper sulfate, the main ingredient of the electrolyte. Such low molecular glue is also receiving attention for its influence on the physical properties of copper foil, etc., and development of a technique for its measurement has been keenly sought for.

### SUMMARY OF THE INVENTION

An object of the present invention is to provide an effective method of measuring concentrations and molecular weight distributions of glue and gelatin, particularly low molecular weight (<2500) glue and gelatin, dissolved in solution.

As a result of researches, the present inventors have found that the above object is accomplished by a combination of high performance liquid chromatography (hereinafter "HPLC") and a column-switching technique.

Based on the above finding, the present invention provides a method of measuring a concentration and a molecular weight distributions of glue or gelatin contained in an electrolyte, which comprises HPLC combined with column switching.

The method of the present invention makes it possible to determine the concentration and molecular weight distribution of glue or gelatin, particularly low molecular weight glue or gelatin, in a solution, which will be highly beneficial for creation of optimum conditions of electrolysis or electroplating thereby achieving effective control on physical properties of copper foil produced, such as elongation, tensile strength, roughness and the like.

### BRIEF DESCRIPTION OF THE DRAWINGS

Fig. 1 is a flow chart showing an example of the measuring method of the present invention.
Fig. 2 is a schematic illustration showing an apparatus used in the measuring method of the present invention.
Fig. 3 is a chromatogram of the electrolyte while used in copper electrorefining in Example 1.
Fig. 4 is the molecular weight distribution of glue obtained from the chromatogram of Fig. 3.
Fig. 5 is a chromatogram of the copper electrolyte in Example 2.
Fig. 6 is a glue molecular weight calibration curve used in Example 2.
Fig. 7 is a graph showing the relationship between glue concentration and copper foil tensile strength in Test Example 1.

### DETAILED DESCRIPTION OF THE INVENTION

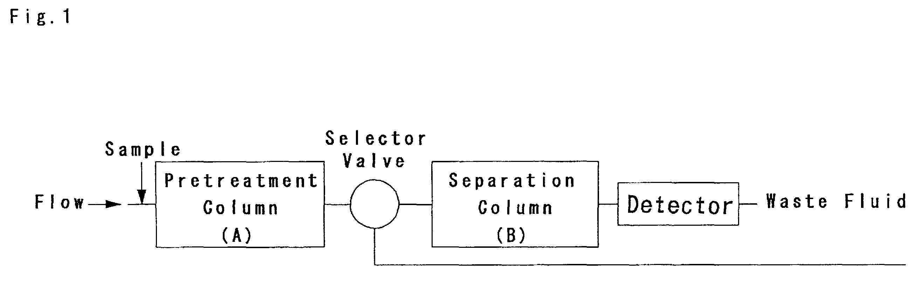

In the present invention the concentration and molecular weight distribution of glue present in a solution, particularly an electrolyte, are measured by use of HPLC, especially gel-permeation chromatography (hereinafter "GPC"). An example of the method of measurement is shown in the flow chart of Fig. 1.

In Fig. 1, a glue-containing solution is used as a sample. In particular, a copper electrolyte used in copper foil manufacturing is used preferably. Copper electrolytes for other purposes and other electrolytes are also useful. The measuring method is applicable to a copper plating bath or other plating baths as well.

In a preferred embodiment a mixed solution comprising 3% by volume or more of acetonitrile and 97% by volume or less of dilute sulfuric acid having a concentration of 0.002 to 0.01 M is used as a mobile phase. By use of this mobile phase, glue is prevented from being adsorbed by an adsorbent of size exclusion mode in the column, thereby securing accurate measurement. If the acetonitrile content is less than 3 vol%, the effect in preventing glue adsorption is insufficient. This effect holds good at considerably higher acetonitrile content, but too high an acetonitrile content adversely affects the adsorbent of size exclusion mode in the column. A preferred acetonitrile content is from 5 to 20% by volume.

It is desirable to use as a pretreatment column (A) a column packed with a filler of size exclusion mode whose exclusion limit is 2500 or smaller. In the pretreatment column (A) glue is separated from the electrolyte component of the sample (electrolyte), and the glue is further sent to a separation column (B), while the electrolyte component is discharged out of the system. Suitable examples of the pretreatment column (A) include a polyether ether ketone (hereinafter "PEEK") column of 7.5 mm in inner diameter and 250 mm in length packed with Sephadex G-15 (particle size: 66 µm or smaller; exclusion limit: 1500; available from Pharmacia Biosystems).

Since solutes in a sample which are bigger than the maximum pore size of the filler are not caught by the filler, i.e., excluded from adsorption, they are not separated from matrix components of the sample. The limit of the excluded molecular weight is called an exclusion limit, which represents the upper limit of molecular weights that can be separated from the matrix on a GPC column.

It is preferred to use a filler of size exclusion mode having an exclusion limit of 10000 or greater in the separation column (B). While passing through the separation column (B), glue in the solution is developed according to the molecular weight and eluted in descending order of molecular weight. Examples of suitable separation columns include SHODEX PROTEIN KW-802.5 (exclusion limit: 50000; inner diameter: 8 mm; length: 300 mm; available from Showa Denko K.K.), Asahipak GS-320HQ (exclusion limit: 40000; inner diameter: 7.6 mm; length: 300 mm; available from Showa Denko), and OHpak SB-803HQ (exclusion limit: 100000; inner diameter: 8 mm; length: 300 mm; available from Showa Denko).

It is preferable to use two or more separation columns connected in series to increase separation efficiency of glue from copper ions and sulfate ions. Fillers which can be used in the separation column(s) include silica, carboxylated polyvinyl alcohol, and polyhydroxymethacrylates.

The glue eluted from the separation column(s) is detected in a detector, such as an absorbance detector. The concentration and the average molecular weight of the eluted glue can be calculated from the peak area and the elution time, respectively. More specifically, the concentration is determined by use of a calibration curve prepared from peak areas obtained by analyzing aqueous glue solutions having known concentrations under the same conditions. The molecular weight distribution is determined by use of a calibration curve presenting the relationship between molecular weight and elution time which is prepared by carrying out the same analyses on standard proteins whose molecular weights are known and plotting the elution time against the molecular weight.

The present invention method of measuring the concentration and molecular weight distribution of glue in an electrolyte will further be described in detail. As already mentioned, the method is constructed of a combination of HPLC and column switching. The method is preferably carried out in the following manner.
(a) An apparatus which can be used in carrying out the measurement comprises a solution delivery pump; a six-way valve; an injector provided between, and connected to, the solution delivery pump and the first port of the six-way valve; a pretreatment column connected to the second port of the six-way valve; a first detector provided between, and connected to, the pretreatment column and the fifth port of the six-way valve; a separation column connected to the sixth port of the six-way valve; a second detector provided between, and connected to, the separation column and the third port of the six-way valve; a data processor for obtaining the concentration and molecular weight distribution of glue based on the information from the second detector; a discharge pipe connected to the forth port of the six-way valve; and a thermostat for maintaining the pretreatment column and the separation column at a constant temperature.
(b) A mixed solution comprising 3% by volume or more of acetonitrile and 97% by volume or less of dilute sulfuric acid is used as a mobile phase.
(c) A filler of size exclusion mode whose exclusion limit is 2500 or smaller is used as a filler of the pretreatment column.
(d) A filler of size exclusion mode having an exclusion limit of 10000 or greater is used as a filler of the separation column.
(e) A small amount of an electrolyte containing glue is put in the injector and sent to the pretreatment column.
(f) In the pretreatment column, the electrolyte is separated into glue and the electrolyte component. The glue is forwarded to the separation column, while the electrolyte component is discharged out of the system.
(g) In the separation column, glue is developed according to the molecular weight and detected in the second detector. The detection data are processed in the data processor to obtain the glue concentration and molecular weight distribution.

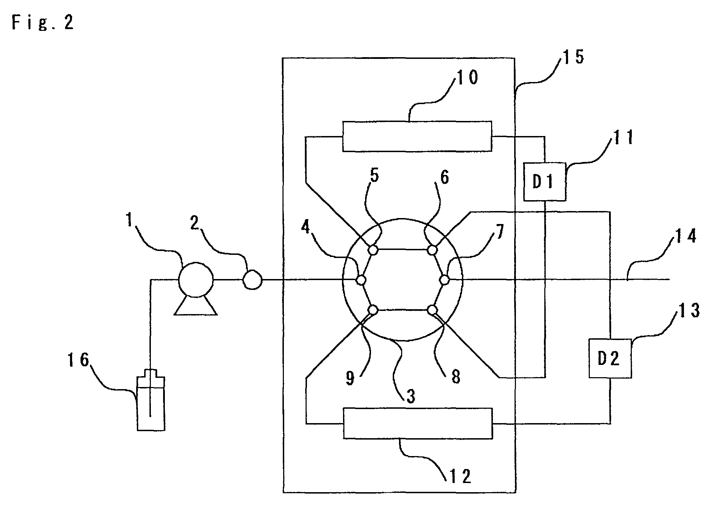

The measuring method of the present invention is further described with reference to Fig. 2, which schematically shows an apparatus used in the method of the present invention. The apparatus shown comprises a solution delivery pump 1; a six-way valve 3; an injector 2 which is provided between, and connected to, the solution delivery pump 1 and the first port 4 of the six-way valve 3; a pretreatment column 10 connected to the second port 5 of the six-way valve 3; a first detector 11 provided between, and connected to, the pretreatment column 10 and the fifth port 8 of the six-way valve 3; a separation column 12 connected to the sixth port 9 of the six-way valve 3; a second detector 13 provided between, and connected to, the separation column 12 and the third port 6 of the six-way valve 3; a data processor (not shown) for obtaining the concentration and molecular weight distribution of glue based on the information from the second detector 13; a discharge pipe 14 connected to the fourth port 7 of the six-way valve 3; a thermostat 15 for maintaining the pretreatment column 10 and the separation column 12 at a constant temperature; and a mobile phase tank 16. These units are connected through piping using PEEK- or Teflon-made tubes.

A mobile phase comprising a phosphoric acid buffer as a pH buffer and sodium chloride as a neutral salt is known as a general mobile phase for size exclusion chromatography. However, if a glue-containing electrolyte is subjected to size exclusion chromatography using this mobile phase, part of the glue is adsorbed by the filler of size exclusion mode in the column, resulting in a failure to make an accurate measurement. When, on the other hand, a mixed solution comprising 97 vol% or less of dilute sulfuric acid and 3 vol% or more of acetonitrile is used as a mobile phase, glue is prevented from being adsorbed by the filler of size exclusion mode thereby achieving accurate measurement.

The aim of providing the pretreatment column is to remove the electrolyte component co-existing in an electrolyte. The object of measuring glue concentration and molecular weight distribution is to know the amount of the glue whose molecular weight is in a range effective for the purpose of addition of glue to an electrolyte. Accordingly, the exclusion limit of the filler of size exclusion mode to be used in the pretreatment column is decided by the lower limit of the effective molecular weight range of glue and the molecular weight of the co-existing electrolyte component. As previously noted, the exclusion limit of the size-exclusion mode filler in the pretreatment column is generally 2500 or smaller, e.g., 1500.

The aim of using the separation column is to measure the concentration and the molecular weight distribution of glue existing in the electrolyte in a small amount. Accordingly, the exclusion limit of the size-exclusion mode filler used in the separation column depends on the upper limit of the effective molecular weight range of glue in the electrolyte. As already described, the exclusion limit of the size-exclusion mode filler in the separation column is generally 10000 or greater, e.g., 50000.

The detectors which can be used in the present invention include those commonly employed in HPLC, with which glue can be detected on the level of milligram per liter, for example, an absorbance detector.

The data processor which can be used in the invention is not particularly limited and includes any data processors equipped with a computing function for obtaining glue concentrations and molecular weight distributions based on the information from the detector.

In the beginning of the measurement by use of the apparatus described above, the first port 4 and the second port 5 of the six-way valve 3 are connected, the fifth port 8 and the sixth port 9 are connected, and the third port 6 and the fourth port 7 are connected. In this state a mobile phase consisting of, for example, 0.005 M sulfuric acid and acetonitrile at a volume ratio of 95:5 is made to flow from the mobile phase tank 16 by the solution delivery pump 1 in the order of injector 2 → six-way valve 3 → pretreatment column 10 → first detector 11 → six-way valve 3 → separation column 12 → second detector 13 → six-way valve 3 → discharge pipe 14. While the mobile phase is flowing through the system, 200 µl of an electrolyte containing glue, either as such or as diluted with pure water to make 200 µl, is introduced into the injector 2. The electrolyte flows into the pretreatment column 10 packed with an aqueous size-exclusion mode filler having an exclusion limit of 2500 or less, for example, a PEEK-made column having an inner diameter of 7.5 mm and a length of 250 mm packed with Sephadex G-15 (exclusion limit: 1500; available from Pharmacia Biosystems), where the electrolyte is separated by the separation principle of size exclusion chromatography. As a result, glue having higher molecular weights is first eluted, and elution of the low-molecular electrolyte component follows.

The glue and the electrolyte component eluted from the pretreatment column 10 are monitored by the first detector 11, for example, an absorbance detector at a wavelength of 210 nm. After the glue is introduced into the separation column 12 and before a large quantity of the electrolyte component enters the separation column 12, the six-way valve 3 is switched to connect the first port 4 to the sixth port 9, the third port 6 to the second port 5, and the fifth port 8 to the fourth port 7, respectively. In this switched-over state, the mobile phase flows in the order of tank 16 → injector 2 → sixth-way valve 3 → separation column 12 → second detector 13 → sixth-way valve 3 → pretreatment column 10 → first detector 11 → six-way valve 3 → discharge pipe 14, and the electrolyte component is discharged out of the system through the pipe 14.

As the separation column 12, a column packed with an aqueous size-exclusion mode filler having an exclusion limit of 10000 or more, for example, SHODEX PROTEIN KW-802.5 (exclusion limit: 50000; inner diameter: 8 mm; length: 300 mm; available from Show Denko) is used. The glue introduced into the separation column 12 is developed according to the molecular weight and the molecular weight distribution and then eluted. The eluted glue is detected by the second detector, for example, an absorbance detector at a wavelength of 210 nm. The glue concentration and the molecular weight distribution are calculated by the data processor based on the detection data.

According to the present invention, glue can be separated from the electrolyte component automatically while flowing in the stream of the mobile phase in a pretreatment column which is provided in front of a separation column. This eliminates the need to preliminarily treat the electrolyte before introduction into a measurement system and, as a result, decomposition of glue during measurement can be reduced to the minimum. Since the electrolyte component which co-exists in a large amount is discharged out of the measurement system by switching the six-way valve, the influences of the co-existing substance can be reduced. The operation of the separation column furnishes information on the molecular weight distribution as well as the concentration of glue, which makes it possible to monitor the glue decomposition progress with time.

The method of the present invention enables us to measure the concentration and molecular weight distribution of glue present in a small amount (on the level of milligram per liter) in various electrolytes and plating baths. In analyzing a copper electrolyte, for example, the method makes it feasible to measure the concentration and molecular weight distribution of glue having a molecular weight as low as 790 or more. This will reveal the influences of low molecular (e.g., 790 to 2500) glue components that have heretofore been unmeasurable upon the physical properties of electro-deposited copper foil, such as high-temperature elongation, roughness, and tensile strength. The information thus revealed will be made use of for process control. When the method of the invention is applied to other electrolytes, plating baths, etc., alterations should be made in some cases to the mobile phase composition. The kinds of the pretreatment column and the separation column are also subject to variation and are preferably selected according to the molecular size of the glue in a sample.

The present invention will now be illustrated in greater detail with reference to Examples and Test Example, but it should be understood that the invention is not construed as being limited thereto.

### EXAMPLE 1

A sample was taken from a copper electrolyte while used in copper electrorefining and, immediately after sampling, 2-fold diluted with pure water. The sample (as diluted) was kept in a refrigerator until analysis.

The apparatus shown in Fig. 2 was used for analysis, in which:
Mobile phase: 0.005 M sulfuric acid/acetonitrile=95:5 by volume
Pretreatment column: PEEK-made column (inner diameter: 7.5 mm; length: 250 mm) packed with Sephadex G-15 (particle size: ≤66 µm; exclusion limit: 1500; available from Pharmacia Biosystems)
Separation column: SHODEX PROTEIN KE-802.5 (exclusion limit: 50000; inner diameter: 8 mm; length: 300 mm; available from Showa Denko)

The two columns were maintained at 25°C. The mobile phase was delivered through the system at a constant flow rate of 0.6 mm/min and, when the base line became steady, 200 µl of the sample was injected into the injector and introduced into the pretreatment column. Glue was eluted first. When Cu²⁺ ions began to be eluted, the six-way valve was switched so that the electrolyte component which was eluted thereafter was discharged out of the system. The flow containing the eluted glue and later containing Cu²⁺ ions was then introduced into the separation column, where the glue was separated according to the molecular weight. The eluted glue was detected in an absorbance detector at a measuring wavelength of 210 nm. The signals from the detector were put into the memory of a data processor capable of GPC computation to obtain the molecular weight distribution and the concentration with the aid of previously prepared molecular weight calibration curve and concentration curve. Various average molecular weights, such as a number average molecular weight and a weight average molecular weight, can be calculated from the molecular weight distribution curve thus obtained.

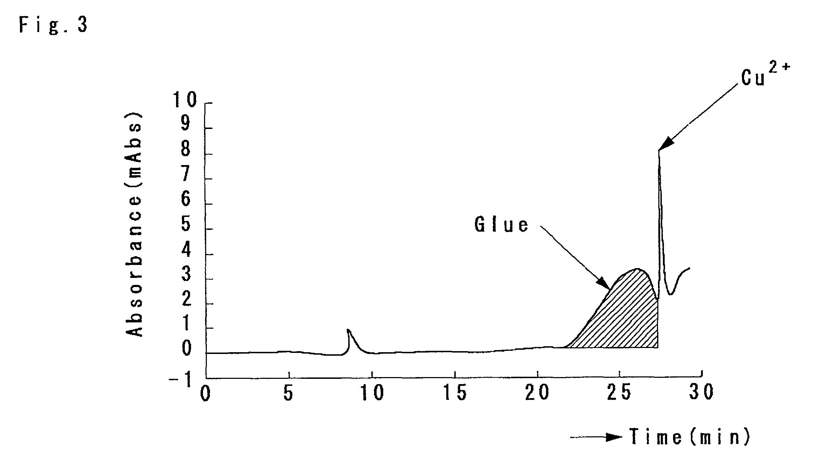

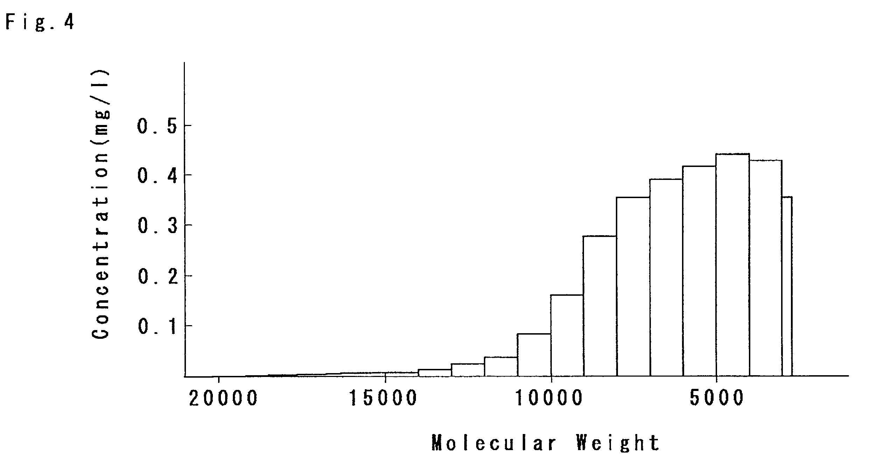

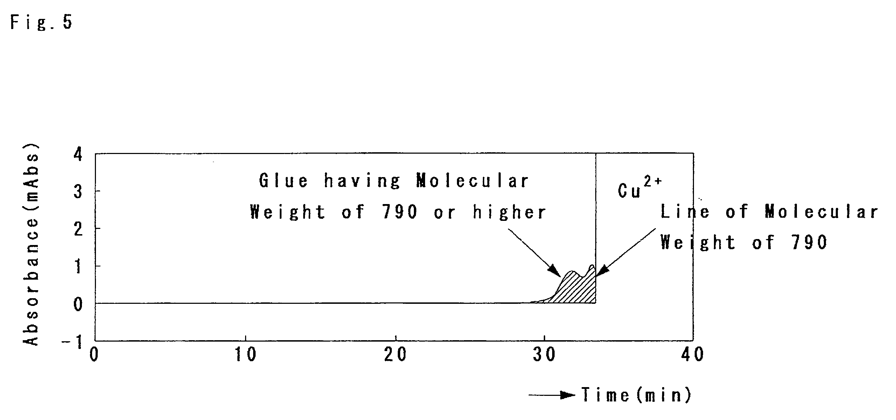

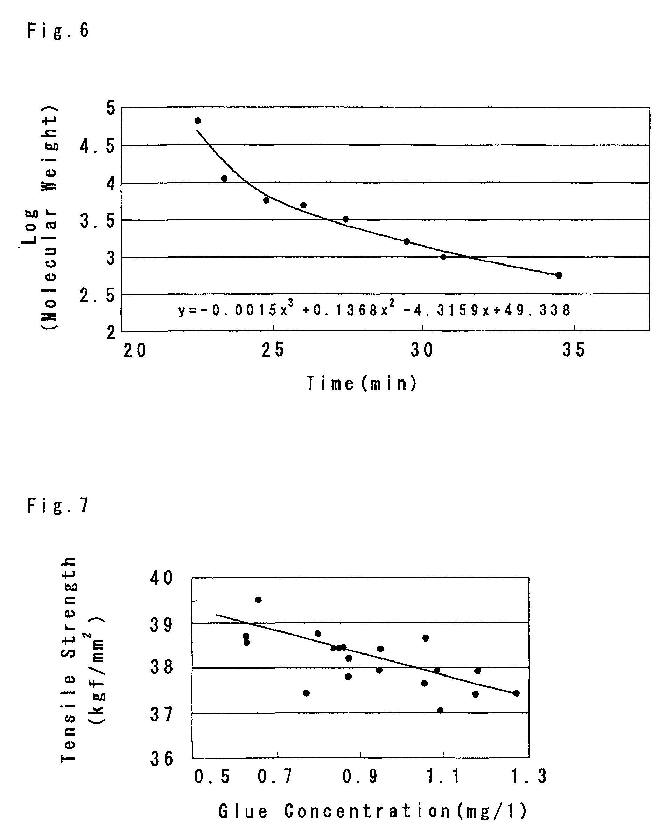

The chromatogram obtained from the analysis on the sample is shown in Fig. 3. The molecular weight distribution of the glue obtained from the chromatogram is shown in Fig. 4. The concentration of glue having a molecular weight of about 2500 or higher which was obtained from the peak area of glue immediately before elution of Cu²⁺ ions was found to be 2.8 mg/l.

### EXAMPLE 2

Measurement of the molecular weight of glue in a copper electrolyte was carried out under the following conditions. The resulting chromatogram is shown in Fig. 5. Calculations for obtaining the molecular weights were based on the calibration curve shown in Fig. 6. The concentration of glue having molecular weights of 790 or higher which was obtained from the peak area of glue immediately before elution of Cu²⁺ ions was 0.9 mg/l.

### Measuring conditions:

Pretreatment column: PEEK-made column (inner diameter: 7.5 mm; length: 250 mm) packed with Sephadex G-15 (particle size: ≤66 µm; exclusion limit: 1500; available from Pharmacia Biosystems)
Separation column: Asahipak GS-320HQ (exclusion limit: 40000; inner diameter: 7.6 mm; length: 300 mm; available from Showa Denko)
Temperature: 25°C
Mobile phase: 0.005 M sulfuric acid/acetonitrile=80/20 by volume
Flow rate of mobile phase: 0.6 ml/min
Amount of injected sample: 200 µl
Detection: UV absorption at 210 nm

As is apparent from the chromatogram of Fig. 5, the measuring method of the present invention makes it feasible to determine the concentration and molecular weight distribution of glue having a molecular weight of 790 or higher.

### TEST EXAMPLE 1

Copper foil having a thickness of 35 µm was produced by electrolysis of an electrolyte containing 80 g/l of copper, 150 g/l of free sulfuric acid, 3 mg/l of chloride ions, and a varied amount (mg/l) of glue at a liquid temperature of 49°C and a current density of 100 A/dm².

The glue concentration in the electrolyte was monitored by the method of the present invention during the electrolysis. The relationship between the glue concentration and tensile strength of the resulting copper foil is shown in Fig. 7. It can be seen from Fig. 7 that the tensile strength of copper foil is inversely proportional to the glue concentration. Now that the present invention provides a method for accurately measuring the glue concentration, it is feasible to control the tensile strength of copper foil arbitrarily.

## Claims

1. A method of measuring a concentration and a molecular weight distribution of glue or gelatin contained in an electrolyte, which comprises high performance liquid chromatography combined with column switching.

2. The method according to claim 1, wherein said high performance liquid chromatography is gel-permeation chromatography.

3. The method according to claim 1, wherein a mixed solution comprising 3% by volume or more of acetonitrile and 97% by volume or less of dilute sulfuric acid having a concentration of 0.002 to 0.01 M is used as a mobile phase, a column packed with a filler of size exclusion mode whose exclusion limit is 2500 or smaller is used as a pretreatment column, and a column packed with a filler of size exclusion mode whose exclusion limit is 10000 or greater is used as a separation column.

4. The method according to claim 3, wherein a plurality of said separation columns are used in a series.

5. The method according to claim 3, wherein said filler of said separation column is selected from silica, carboxylated polyvinyl alcohol and a polyhydroxymethacrylate.
